(19) Europäisches Patentamt
European Patent Office
Office européen des brevets

(11) Veröffentlichungsnummer: **0 097 127**
**A2**

(12) # EUROPÄISCHE PATENTANMELDUNG

(21) Anmeldenummer: **83810255.6**

(22) Anmeldetag: **10.06.83**

(51) Int. Cl.³: **C 07 D 501/00**, A 61 K 31/545

(30) Priorität: **16.06.82 CH 3726/82**

(43) Veröffentlichungstag der Anmeldung: **28.12.83 Patentblatt 83/52**

(84) Benannte Vertragsstaaten: **AT BE CH DE FR GB IT LI LU NL SE**

(71) Anmelder: **CIBA-GEIGY AG, Patentabteilung Postfach, CH-4002 Basel (CH)**

(72) Erfinder: **Fechtig, Bruno, Dr., Hinterlindenweg 1, CH-4153 Reinach (CH)**

(54) **Cephalosporinverbindungen, Verfahren zu ihrer Herstellung und sie enthaltende pharmazeutische Präparate.**

(57) Die Erfindung betrifft neue unter physiologischen Bedingungen spaltbare substituierte Carboxymethylester des Ceftizoximes, Verfahren zu ihrer Herstellung, pharmazeutische Präparate, die solche Verbindungen enthalten, und ihre Verwendung als antibakterielle Antibiotika.

EP 0 097 127 A2

BEZEICHNUNG GEÄNDERT
siehe Titelseite

Neue Carboxymethylester

Die Erfindung betrifft neue, unter physiologischen Bedingungen spaltbare substituierte Carboxymethylester des Ceftizoximes, Verfahren zu ihrer Herstellung und pharmazeutische Präparate, die solche Verbindungen enthalten.

Im folgenden soll unter Ceftizoxime die freie 7β-[2-(2-Amino-4-thiazolyl)-2-(syn)-methoxyiminoacetamido]-3-cephem-4-carbonsäure verstanden werden. Diese Säure, das Natriumsalz davon, das in der Literatur ohne es als Natriumsalz zu spezifieren, gelegentlich gleichfalls nur als Ceftizoxime bezeichnet wird, einige weitere Salze und auch Ester, einschliesslich einiger physiologisch spaltbarer Ester, sind aus der deutschen Offenlegungsschrift 28 10 922, der britischen Patentanmeldung 2.052.933 und aus der europäischen Patentanmeldung 8343 bekannt. Diese Verbindungen sind wertvolle, antibiotisch wirksame Substanzen, die insbesondere als antibakterielle Antibiotika verwendet werden können. Während die Salze nur zur parenteralen Applikation geeignet sind, haben die unter physiologischen Bedingungen spaltbaren Ester den Vorteil, auch nach oraler Applikation wirksam zu sein.

Es besteht weiterhin ein Bedürfnis nach oral verabreichbaren Verbindungen dieses Types, die gegenüber den bekannten, oral wirksamen Estern des Ceftizoximes ähnliche oder verbesserte Eigenschaften aufweisen. Ueberraschenderweise wurde gefunden, dass gewisse substituierte Carbonylmethylester vom Ceftizoxime bei oraler Anwendung gute antibiotische Wirksamkeit besitzen. Die orale Wirksamkeit der Ester der

vorliegenden Erfindung ist insofern überraschend, als viele andere substituierte Carbonylmethylester bei oraler Applikation keine oder nur geringe Wirksamkeit besitzen.

Die vorliegende Erfindung betrifft die neuen Verbindungen der Formel

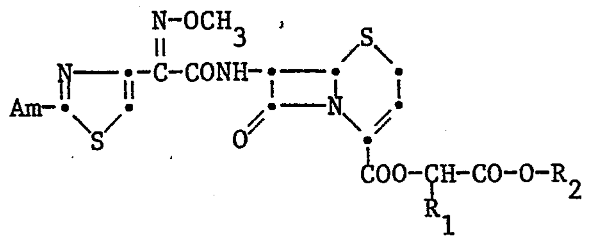

I,

worin $R_1$ Wasserstoff oder Niederalkyl, $R_2$ einen cycloaliphatischen Rest und Am eine gegebenenfalls geschützte Aminogruppe bedeuten, Säureadditionssalze von solchen Verbindungen mit salzbildender Gruppe, Verfahren zu ihrer Herstellung, pharmazeutische Präparate, die solche Verbindungen enthalten und ihre Verwendung.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Gruppen, sofern nicht ausdrücklich anders definiert, bis zu 7, bevorzugt bis zu 4 C-Atome enthalten.

Eine Niederalkylgruppe $R_1$ ist insbesondere $C_1$-$C_4$-Niederalkyl, wie Methyl, Aethyl, Propyl, Isopropyl, 2-Methylpropyl oder Butyl. Bevorzugt ist $R_1$ Methyl und insbesondere Wasserstoff.

Ein cycloaliphatischer Rest ist ein mono- oder poly-, z.B. bi- oder tricyclischer, gesättigter oder ungesättigter, unsubstituierter oder substituierter Kohlenwasserstoffrest mit z.B. bis zu 14, bevorzugt bis zu 10, oder in substituiertem Zustand auch noch mehr, z.B. bis zu 20 Kohlenstoffatomen.
Entsprechende monocyclische Kohlenwasserstoffreste enthalten z.B. 3 bis 8 C-Atome, sind in gesättigter Form z.B. Cyclopropyl, Cyclobutyl, Cyclopentyl, Cyclohexyl, Cycloheptyl oder Cyclooctyl, oder in ungesättigter Form z.B. 1- oder 2-Cyclopropen-1-yl, 1-,2- oder 3-Cyclopenten-1-yl,

1,3-, 1,4- oder 2,4-Cyclopentadien-1-yl, 1-, 2- oder 3-Cyclohexen-1-yl, 1,3-, 1,4-, 2,4- oder 2,5-Cyclohexadien-1-yl, oder 2-Cyclohepten-1-yl. Polycyclische Kohlenwasserstoffreste haben insbesondere 6-10 C-Atome und sind in gesättigter Form z.B. Bicyclohexyl, Bicycloheptyl, Bicyclooctyl, Bicyclononyl, Bicyclodecyl oder Tricyclodecyl, z.B. Bicyclo[3.1.0]hex-1-, -2- oder -4-yl, Bicyclo[4.1.0]hept-1- oder -4-yl, Bicyclo[2.2.1]hept-2-yl, z.B. endo- oder exo-Norbornyl, Bicyclo[3.2.1]-oct-2-yl, Bicyclo[3.3.0]oct-3-yl, Bicyclo[3.3.1]non-9-yl, Tricyclo-[5.2.1.$0^{2,6}$]dec-8-yl oder Adamantyl, oder in ungesättigter Form entsprechende Bicycloalkenyle, z.B. Bicyclo[2.2.1]hept-5-en-2-yl, z.B. 5-Norbornen-2-yl, Bicyclo[2.2.2]oct-5-en-2-yl oder Hexahydro-4,7-methanoind-1-en-6-yl.

Substituenten der cycloaliphatischen Kohlenwasserstoffreste sind Hydroxy, veräthertes Hydroxy, z.B. Alkoxy oder Alkenyloxy mit bis zu etwa 20 C-Atomen, insbesondere Niederalkoxy, z.B. Methoxy, Aethoxy, Propoxy, Butoxy oder tert.Butoxy, Decyloxy, Dodecyloxy, Eikosyloxy oder Allyloxy, verestertes Hydroxy, z.B. Alkanoyloxy oder Alkenoyloxy mit bis zu 20 C-Atomen, z.B. Acetoxy, Propionyloxy, Butyryloxy, Decanoyloxy, Dodecanoyloxy, Hexadecanoyloxy, Eikosanoyloxy oder Octadec-9-enoyloxy, Halogen, z.B. Fluor oder Chlor, Amino, Mono- oder Dialkylamino, insbesondere Mono- oder Diniederalkylamino, z.B. Methyl-, Dimethyl-, Aethyl-, Diäthyl-, Isopropyl-, Diisopropyl-, Aethyl-isopropyl- oder Dibutylamino, Mono- oder Di-Acylamino, insbesondere Mono- oder Diniederalkanoylamino, z.B. Acetyl- oder Diacetylamino, Carboxy, funktionell abgewandeltes, z.B. verestertes oder amidiertes Carboxy, z.B. Alkyloxycarbonyl, worin Alkyl bis zu 20 C-Atome enthält, insbesondere Niederalkoxycarbonyl, z.B. Methoxy-, Aethoxy-, Propoxy- oder Butoxycarbonyl, Carbamoyl oder Nitril und insbesondere Niederalkyl, z.B. Methyl, Aethyl, Propyl und Isopropyl, Niederalkenyl, z.B. 1-Propen-2-yl, oder Niederalkyliden, z.B. Methyliden oder Isopropyliden. Von diesen Substituenten können unabhängig voneinander 1-3, oder auch z.B. im Falle von Halogen, bis zu 6 vorhanden sein. Weitere Substituenten sind Phenyl und Cycloaliphatyl-, z.B. Cyclohexylreste, von denen ebenfalls mehrere, z.B. bis zu 3, vorhanden sein können. Bevorzugt sind diese Substituenten Halogen, Hydroxy und Niederalkyl, z.B. Methyl und Isopropyl.

Entsprechende substituierte cycloaliphatische Kohlenwasserstoffreste sind beispielsweise gegebenenfalls veräthertes oder verestertes Hydroxycyclopentyl, Hydroxycyclohexyl, Hydroxycycloheptyl oder Hydroxycyclooctyl, worin die Hydroxygruppen gegebenenfalls durch Niederalkyl, z.B. Methyl, veräthert oder durch Niederalkanoyl, z.B Acetyl, verestert sein können, z.B. 2- oder 3-Hydroxycyclopent-1-yl, 2- oder 3-Methoxycyclopent-1-yl, 2- oder 3-Acetoxycyclopent-1-yl, 2-, 3- oder 4-Hydroxy-, 2-, 3- oder 4-Methoxy- oder 2-, 3- oder 4-Acetoxycyclohex-1-yl, oder 5-Hydroxy-, 5-Methoxy- oder 5-Acetoxycyclooct-1-yl, oder solche Reste die anstelle der Hydroxygruppe Halogen, z.B. Chlor oder Fluor, oder Niederalkyl, z.B. Methyl tragen, Diniederalkylcycloalkyl, beispielsweise Diniederalkylcyclohexyl, z.B. 2,4-, 2,5-, 2,6-, 3,4- oder 3,5-Dimethylcyclohexyl, Menthanyl (Methyl-isopropylcyclohexyl), z.B. p-Menthan-2-yl, insbesondere (ℓ)-Menthyl, oder m-Menthan-3-yl, Menthenyl, z.B. 1-p-Menthen-3-yl, 3-p-Menthen-1-yl, 8-p-Menthen-1-yl, oder 8-p-Menthen-3-yl, Thujanyl (1-Isopropyl-4-methylbicyclo[3.1.0$^{1,5}$]-hexyl), z.B. 3-Thujanyl, Thujenyl, z.B. 4(10)-Thujen-3-yl (Sabinyl), Caranyl (3,7,7-Trimethylbicyclo[4.1.0$^{1,6}$]heptyl), z.B. 4-Caranyl, Carenyl, z.B. 4-Caren-3-yl, Pinanyl(2,6,6-Trimethylbicyclo[3.1.1]-heptyl),z.B. 3-Pinanyl, Pinenyl, z.B. 3-Pinen-2-yl, Bornanyl(3,7,7-Trimethylbicyclo[2.2.1]heptyl), z.B. 2-Bornanyl, insbesondere d-, ℓ- oder d,ℓ-Bornyl, Bornenyl, z.B. 4-Bornen-2-yl, oder 2,2-Dimethylbicyclo[2.2.1]hept-3-yl, oder Di- oder Triniederalkyltricycloalkyl, z.B. 3,8-Dimethylhexahydro-4,7-methano-ind-1-en-6-yl oder 3,5,7-Trimethyl-1-adamantyl.

Das Kohlenstoffatom, an das $R_1$ gebunden ist, und die in $R_2$ vorhandenen optisch aktiven C-Atome können die R-, S- oder R,S-Konfiguration besitzen.

In einer geschützten Aminogruppe Am ist die Schutzgruppe eine der in der Penicillin- , Cephalosporin- und Peptidchemie verwendeten.

Eine solche Schutzgruppe ist leicht, das heisst ohne dass unerwünschte Nebenreaktionen stattfinden, beispielsweise solvolytisch, reduktiv oder photolytisch, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965, sowie in "Methoden der organischen Chemie", Houben-Weyl, 4. Auflage, Bd. 16/1, Georg Thieme Verlag, Stuttgart, 1974.

Eine geschützte Aminogruppe Am kann z.B. in Form einer leicht spaltbaren Acylamino-, Arylmethylamino-, verätherten Mercaptoamino-, 2-Acyl-niederalk-1-en-1-yl-amino-, Silyl- oder Stannylaminogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogen-niederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylreste verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Diphenylniederalkylphosphoryl, z.B. Dibenzylphosphoryl oder Di-4-nitrobenzylphosphoryl, gegebenenfalls substituiertes Phenyloxy-phenyl-phosphonyl, z.B. Phenyloxyphenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino. Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silyl- oder Stannylaminogruppe ist in erster Linie eine organische Silyl- bzw. Stannylaminogruppe, worin das Silicium- bzw. Zinnatom vorzugweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Line Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl, oder entsprechend substituiertes Stannyl, z.B. Tri-n-butylstannyl.

Eine Aminogruppe kann auch in protonierter Form geschützt werden; als entsprechende Anionen kommen in erster Linie diejenigen von

starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgrpppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Verbindungen der Formel I mit einer basischen Aminogruppe Am können Säureadditionssalze, z.B. mit starken anorganischen Säuren, wie Chlorwasserstoff- oder Bromwasserstoffsäure, Schwefelsäure oder Phosphorsäure, oder mit geeigneten organischen Carbon- oder Sulfonsäuren, z.B. Trifluoressigsäure oder Methansulfonsäure bilden.

Die Methoxyiminogruppe liegt bevorzugt in der syn-Form (Z-Form) vor.

Die Verbindungen der Formel I, worin Am eine freie Aminogruppe ist und ihre pharmazeutisch verwendbaren, nichttoxischen Salze sind wertvolle, antibiotisch wirksame Substanzen, die insbesondere als antibakterielle Antibiotika verwendet werden können. Beispielsweise haben sie in vivo bei oraler Applikation an der Maus bei systemischen Infektionen mit Staphylococcus aureus eine $ED_{50}$ von etwa 70-100 mg/kg, und bei systemischen Infektionen mit Enterobakterien, z.B. Escherichia coli, Klebsiella pneumonia, Proteus mirabilis oder Proteus morganii eine $ED_{50}$ von etwa 0.35 bis 100 mg/kg.

Beispielsweise wurden mit den Testverbindungen

1. Ceftizoxime-(ℓ)-bornyloxycarbonylmethylester
2. Ceftizoxime-cyclohexyloxycarbonylmethylester
3. Ceftizoxime-(ℓ)-menthyloxycarbonylmethylester

folgende Resultate erhalten:

Chemotherapeutische Wirksamkeit gegen systemische Infektionen in Mäusen.

| Infizierende Keime | $ED_{50}$ (mg/kg) 1. | 2. | 3. |
|---|---|---|---|
| Staphylococcus aureus 10B | 70 | >100 | 30-100 |
| Escherichia coli 205 | 1 | 0,5-5 | 4,0 |
| Escherichia coli 2018 | 0,35 | 0,4 | 1,5 |
| Klebsiella pneumonia | 6 | 7 | 4 |
| Proteus mirabilis | 10 | > 30 | ≥30 |
| Proteus morganii | 30 | >100 | 100 |

Die neuen Verbindungen können daher als antibakterielle orale Antibiotika, z.B. in Form von oral applizierbaren pharmazeutischen Präparaten, wie Kapseln, Tabletten, Sirups oder dergleichen, zur Bekämpfung von entsprechenden Infektionen Verwendung finden. Verbindungen der Formel I, worin Am eine geschützte Aminogruppe ist, werden als Ausgangsmaterialien zur Herstellung von antibiotisch wirksamen Verbindungen der Formel I verwendet.

Die physiologisch spaltbaren Ester der vorliegenden Erfindung zeichnen sich durch eine überraschend gute Resorption nach oraler Verabreichung aus, wie durch die hohe renale Ausscheidung nachgewiesen wird. Die physiologische Spaltbarkeit der vorliegenden Ester zur freien Säure Ceftizoxime kann in menschlichem Blut nachgewiesen werden.

Die vorliegende Erfindung betrifft in erster Linie diejenigen Verbindungen der Formel I, worin Am eine freie Aminogruppe, $R_1$ Wasserstoff und $R_2$ gegebenenfalls durch ein bis drei Niederalkylgruppen, z.B. ein bis drei Methyl- und/oder Isopropylgruppen, substituiertes Cycloalkyl oder Bicycloalkyl, z.B. Cyclohexyl, Bicyclohexyl oder Bicycloheptyl, insbesondere ein cyclisches Terpenradical, z.B. ein Menthan-, Menthen-, Thujan-, Thujen-, Caran-, Caren-, Pinan-, Pinen-, Bornan- oder Bornen- oder ein entsprechendes methylfreies Norcaran-, Norpinan- oder Norbornanradical, beispielsweise (ℓ)-Menthyl, (ℓ)-Bornyl oder endo- oder exo-Norbornyl, bedeutet, und ihre pharmazeutisch annehmbaren Salze, sowie die entsprechenden Verbindungen mit geschützten funktionellen Gruppen.

Besonders hervorzuheben sind die Verbindungen der Formel I, worin Am die freie Aminogruppe, $R_1$ Wasserstoff und $R_2$ (ℓ)-Bornyl, Cyclohexyl oder (ℓ)-Menthyl bedeuten, und ihre pharmazeutisch annehmbaren Salze.

Die Erfindung betrifft insbesondere die in den Beispielen beschriebenen Verbindungen der Formel I und deren pharmazeutisch annehmbaren Salze.

Die Verbindungen der vorliegenden Erfindung werden nach an sich bekannten Verfahren hergestellt.

So werden Verbindungen der Formel I hergestellt, indem man
a) die 7β-Aminogruppe in einer Verbindung der Formel

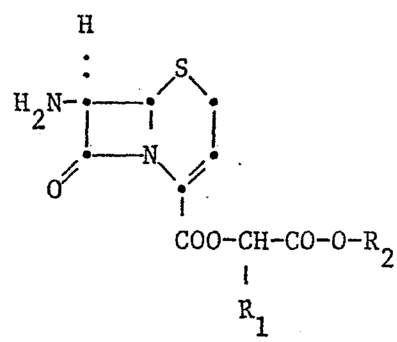

II ,

worin die Aminogruppe gegebenenfalls durch eine die Acylierung erlaubende Gruppe substituiert ist und worin $R_1$ und $R_2$ die unter Formel I gegebenen Bedeutungen haben, durch Behandeln mit einem den Acylrest einer Carbonsäure der Formel

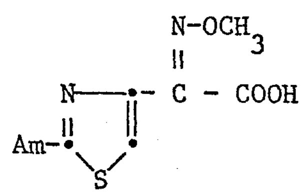

III

einführenden Acylierungsmittel, worin Am die unter Formel I angegebenen Bedeutungen hat, acyliert, oder
b) eine Verbindung der Formel

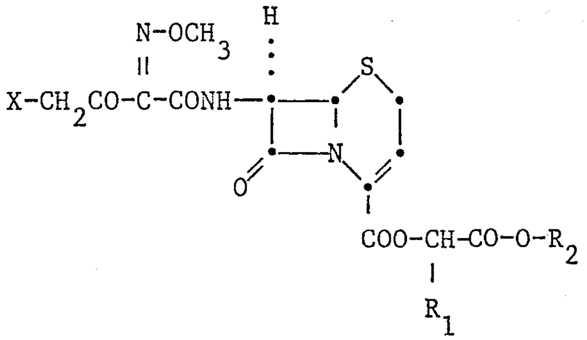

IV ,

worin X Halogen bedeutet, $R_1$ und $R_2$ die unter Formel I genannten Bedeutungen haben, mit einem Thioharnstoff der Formel Am-CS-$NH_2$ oder einem Salz davon kondensiert, oder

c) aus einer Verbindung der Formel

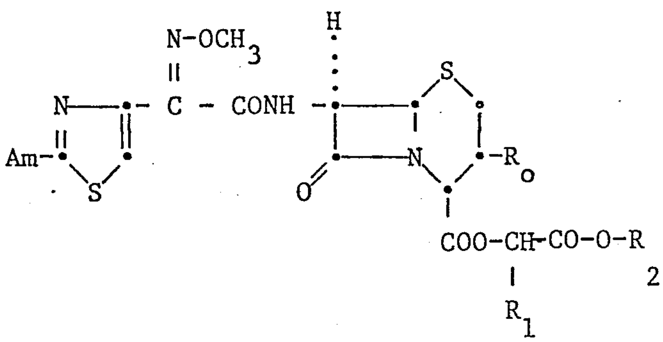

V ,

worin Am, $R_1$ und $R_2$ die unter Formel I angegebenen Bedeutungen haben und $R_o$ Hydroxy, verestertes Hydroxy oder gegebenenfalls substituiertes Amino ist, eine Gruppe H-$R_o$ abspaltet, oder

d) in einer Verbindung der Formel

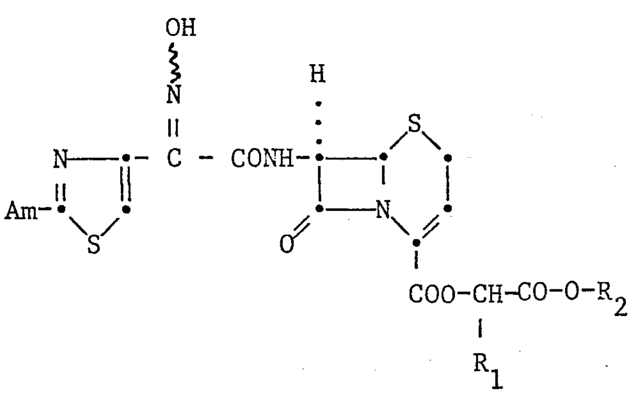

VI ,

worin Am, $R_1$ und $R_2$ die unter Formel I gegebenen Bedeutungen haben, die Hydroxyiminogruppe methyliert, oder

e) in einer Verbindung der Formel

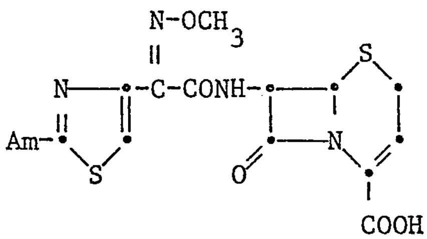

VII ,

worin Am die unter Formel I gegebene Bedeutung hat, die Carboxylgruppe oder ein reaktionsfähiges Derivat davon, mit einem Alkohol der Formel

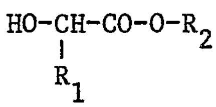

VIII ,

worin $R_1$ und $R_2$ die unter Formel I angegebenen Bedeutungen haben, oder einem reaktionsfähigen Derivat davon, in eine veresterte Carboxylgruppe überführt, oder

f) in einer Verbindung der Formel

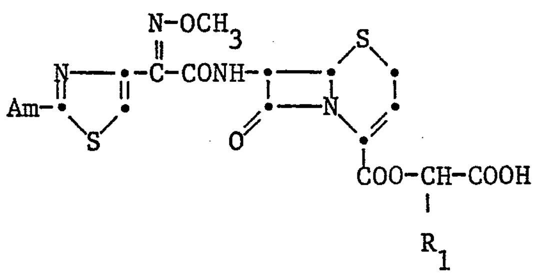

IX ,

worin Am und $R_1$ die unter Formel I gegebenen Bedeutungen haben, die Carboxylgruppe mit einem Alkohol der Formel $R_2$-OH (X) oder einem reaktionsfähigen Derivat davon in eine veresterte Carboxylgruppe überführt, und/oder

g) zur Herstellung einer Verbindung der Formel I, worin Am eine freie Aminogruppe ist und $R_1$ und $R_2$ die unter Formel I gegebenen Bedeutungen haben, aus einer Verbindung der Formel I, worin Am eine geschützte

Aminogruppe bedeutet, und $R_1$ und $R_2$ die unter Formel I gegebenen Bedeutungen haben, die Aminoschutzgruppe abspaltet und durch Wasserstoff ersetzt, wenn erwünscht, eine erhaltene Verbindung worin Am eine freie Aminogruppe ist in ein Salz oder ein erhaltenes Salz in eine freie Verbindung oder in ein anderes Salz überführt und/oder, wenn erwünscht, ein erhaltenes Isomerengemisch in die einzelenen Isomeren auftrennt.

Verfahren a): Gegebenenfalls vorhandene, die Aminogruppe substituierende und deren Acylierung erlaubende Reste in einem Ausgangsmaterial der Formel II sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen.

Die genannten Ylidengruppen sind in erster Linie Arylmethylengruppen, worin Aryl insbesondere für einen carbocyclischen, in erster Linie monocyclischen Arylrest, z.B. für gegebenenfalls durch Nitro oder Hydroxy substituiertes Phenyl steht; solche Arylmethylengruppen sind z.B. Benzyliden, 2-Hydroxybenzyliden oder 4-Nitrobenzyliden, ferner gegebenenfalls, z.B. durch Carboxy, substituiertes Oxacycloalkyliden, z.B. 3-Carboxy-2-oxacyclohexyliden.

Den Acylrest einer Carbonsäure der Formel III einführende Acylierungsmittel sind beispielsweise die Carbonsäure selbst oder reaktionsfähige funktionelle Derivate davon.

Falls eine freie Säure der Formel III, worin alle funktionellen Gruppen ausser der reagierenden Carboxylgruppe geschützt sind, als Acylierungsmittel eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, geeignete Carbonyl-

verbindungen, beispielsweise Carbonyldiimidazol, oder Isoxazolinium-salze, beispielsweise N-Aethyl-5-phenyl-isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydrochinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. amidbildendes, funktionelles Derivat einer Säure der Formel III, worin alle funktionellen Gruppen ausser der reagierenden Säuregruppe geschützt sein können, ist in erster Linie ein Anhydrid einer solchen Säure, inklusive und vorzugsweise ein gemischtes Anhydrid, aber auch ein symmetrisches Anhydrid. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure.

Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere reaktionsfähige Säurederivate einer Säure der Formel III sind aktivierte Ester, wie Ester mit vinylogen Alkoholen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthalyliminoester.

Die Acylierung mit einem Säurederivat, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt. Die reaktionsfähigen Ester, z.B. die 1-Benztriazolester werden auch in Gegenwart eines der genannten Carbodiimide, z.B. N,N-Dicyclohexylcarbodiimid, verwendet.

Die obigen Acylierungen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur, wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

In einer acylierenden Säure der Formel III oder in einem Säurederivat davon kann eine geschützte Aminogruppe auch in ionischer Form vorliegen, d.h. das Ausgangsmaterial der Formel III kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel III mit entsprechend geschützten funktionellen Gruppen, oder eines geeigneten Salzes davon, wie eines Ammoniumsalzes, z.B. mit einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Verfahren b): In einer Ausgangsverbindung der Formel IV ist X Halogen, insbesondere Chlor, aber auch Brom, Jod oder Fluor. Der Thioharnstoff wird in freier Form oder als Salz, insbesondere als Thiolat eines Alkalimetalls, wie Lithium, Natrium oder Kalium, oder auch als Thiolat einer Ammoniumverbindung, in äquivalenter Menge oder in bis zu sechsfachem Ueberschuss, eingesetzt.

Die Reaktion wird im allgemeinen in einem Lösungsmittel, wie Wasser oder einem organischen, nicht-reaktiven Lösungsmittel oder einer Mischung davon, durchgeführt. Als organische Lösungsmittel geeignet sind Alkohole, wie Methanol, Aethanol, Isopropanol, Ketone, wie Aceton, Aether, wie Dioxan oder Tetrahydrofuran, Nitrile, wie Acetonitril, halogenierte Kohlenwasserstoffe, wie Methylenchlorid, Chloroform oder Tetrachlorkohlenstoff, Ester, wie Aethylacetat, oder Amide, wie Dimethylformamid oder Dimethylacetamid, und ähnliche. Die Reaktion kann, falls die freien Verbindungen eingesetzt werden, in Gegenwart einer Base durchgeführt werden. Geeignete Basen sind Alkalimetallhydroxide, wie Natrium- oder Kaliumhydroxid, Alkalimetallcarbonate, wie Natrium- oder Kaliumcarbonat, oder organische tertiäre Stickstoffbasen, wie Triniederalkylamine, z.B. Trimethylamin, Triäthylamin, Aethyl-diisopropylamin, Pyridin und dergleichen. Die Reaktionstemperatur liegt bei Raumtemperatur,oder auch tiefer oder höher, vorzugsweise zwischen -10 bis +100° , insbesondere zwischen 0 bis 40° .

Die Reaktion kann auch stufenweise erfolgen, indem zunächst das ringoffene Zwischenprodukt mit der Teilformel $Am-C(=NH)-S-CH_2-CO-C(=NOCH_3)-$ entsteht, das dann in der zweiten Stufe dehydratisiert wird.

Verfahren c): Die Gruppe $R_o$ in einem Ausgangsmaterial der Formel V kann freies Hydroxy sein, steht aber vorzugsweise für verestertes Hydroxy oder gegebenenfalls substituiertes Amino. Eine veresterte Hydroxygruppe $R_o$ kann durch eine anorganische oder organische Säure, wie eine starke Mineralsäure, z.B. eine Halogenwasserstoffsäure, wie Chlorwasserstoff-, Bromwasserstoff- oder Jodwasserstoffsäure, oder eine organische Carbon- oder Sulfonsäure, inklusive Ameisensäure, wie eine entsprechende aliphatische, cycloaliphatische, cycloaliphatischaliphatische, aromatische, araliphatische, heterocyclische oder heterocyclisch-aliphatische Säure, ferner durch ein Kohlensäurehalbderivat verestert sein. So stellt $R_o$ z.B. Halogen, wie Chlor, Brom oder Jod, Niederalkansulfonyloxy, z.B. Methansulfonyloxy oder Aethansulfonyloxy, Arylsulfonyloxy, z.B. Benzolsulfonyloxy, oder p-Toluolsulfonyloxy, Niederalkanoyloxy, z.B. Acetyloxy oder Propionyloxy, Arylcarbonyloxy, z.B. Benzoyloxy, oder Niederalkoxycarbonyloxy, z.B. Methoxycarbonyloxy oder Aethoxycarbonyloxy, dar. Eine gegebenenfalls substituierte Aminogruppe $R_o$ ist eine primäre, sekundäre oder tertiäre Aminogruppe $-N(R_o^1)$ $(R_o^2)$, worin $R_o^1$ und $R_o^2$ unabhängig voneinander Wasserstoff, oder einen gegebenenfalls substituierten aliphatischen, cycloaliphatischen cycloaliphatischaliphatischen, aromatischen, araliphatischen, heterocyclischen oder heterocyclisch-aliphatischen Rest, oder zusammen eine gegebenenfalls substituierte, gegebenenfalls durch Heteroatome, wie Sauerstoff, Schwefel oder Stickstoff, unterbrochene Polymethylengruppe bedeuten. Solche Aminogruppen $R_o$ sind beispielsweise Amino, Mono- oder Diniederalkylamino, wie Methyl-, Aethyl-, Propyl-, Dimethyl-, Diäthyl-, Dipropyl-, Methyl-äthyl-, oder Methyl-propyl-amino, Mono- oder Dicyclohexylamino, Diphenylamino, Benzyl-, Dibenzyl- oder Phenyläthyl- oder Diphenyläthylamino, Aziridino, Pyrrolidino, Piperidino, Morpholino, Thiomorpholino oder 4-Niederalkylpiperazino.

Die Abspaltung einer Verbindung der Formel $R_o$-H, d.h. von Wasser, einer Säure oder eines Amins, wird vorzugsweise durch Behandeln mit geeigneten Wasser-, Säure- oder Amin-abspaltenden Mittel durchgeführt. Wasser und Amine werden vorzugsweise in Gegenwart eines sauren wasser- bzw. aminabspaltenden Mittels, z.B. einer Säure, vorzugsweise einer starken organischen Carbon- oder Sulfonsäure, wie einer Halogen-niederalkancarbonsäure, z.B. Trifluoressigsäure, oder einer Arylsulfonsäure, z.B. p-Toluolsulfonsäure, eines geeigneten Säurederivats, wie eines Anhydrids oder insbesondere eines Halogenids, wie Chlorids, in erster Linie einer anorganischen, z.B. Phosphor- oder Schwefel-haltigen, Säure, z.B. Phosphoroxychlorid oder Thionylchlorid, wobei man ein solches Derivat, wenn Wasser abgespalten werden soll, üblicherweise in Gegenwart einer Base, wie einer tertiären organischen Base, z.B. Pyridin, verwendet, oder eines geeigneten sauren Ionenaustauschers, wie eines Ionenaustauschers auf Sulfonsäure-Basis, z.B. eines sulfonierten Polystyrolionenaustauschers, abgespalten. Zur Abspaltung von Wasser kann man auch dehydratisierende Carbodiimidverbindungen, z.B. Dicyclohexylcarbodiimid, oder dehydratisierende, über Stickstoffatome disubstituierte Carbonylverbindungen, z.B. Carbodiimidazol, verwenden. Dabei verwendet man diese Mittel üblicherweise in Gegenwart eines Lösungsmittels, wie eines gegebenenfalls halogenierten aliphatischen, cycloaliphatischen oder aromatischen Kohlenwasserstoffs, z.B. Benzol oder Toluol, oder eines Lösungsmittelgemisches, wobei man geeignete saure Mittel, wie Trifluoressigsäure gleichzeitig auch als Lösungsmittel verwenden kann. Wenn notwendig, verwendet man zusätzlich ein wasser-absorbierendes Mittel oder einen Wasserabscheider und arbeitet unter Kühlen oder Erwärmen und/oder in einer Inertgas-, z.B. Stickstoffatmosphäre.

Vorzugsweise bedeutet $R_o$ in einem Ausgangsmaterial der Formel V eine veresterte Hydroxygruppe und man spaltet erfindungsgemäss eine Säure der Formel $H-R_o$ ab. Ueblicherweise verwendet man zu diesem Zweck basische Säure-abspaltende und/oder -neutralisierende Mittel, wie z.B. anorganische Basen, wie verdünnte Alkalimetallhydroxide, z.B. Natrium- oder Kaliumhydroxid, wobei man neben Wasser auch organische Lösungsmittel, wie geeignete Ketone, z.B. Aceton, oder Aether, z.B. Dioxan, oder wässrige Gemische davon verwenden und bei einem pH-Wert von höchstens etwa 9, wenn notwendig, unter Kühlen oder Erwärmen und/oder in einer Inertgas-, z.B. Stickstoffatmosphäre arbeiten kann.

Vorzugsweise verwendet man als Säure-abspaltende Mittel tert.-Amine, insbesondere gute Protonenakzeptoren, die den Lactamring nicht angreifen, in erster Line tert.-aliphatische oder tert.-cycloaliphatische Mono- und Diamine, wie Triniederalkylamine, z.B. Trimethylamin, Triäthylamin oder Aethyl-diisopropylamin, oder bicyclische Diazaverbindungen mit einer Amidin-artigen Anordnung der Ringstickstoffatome, z.B. 1,5-Diazabicyclo [4,3,0]non-5-en oder 1,5-Diazabicyclo[5,4,0]undec-5-en. Ferner kann man basische Ionenaustauscher, z.B. auf Ammoniumhydroxid-Basis, ebenfalls als Säure-abspaltende Mittel einsetzen. Gewisse veresterte Hydroxygruppen $R_o$, insbesondere Sulfonyloxy, z.B. Methylsulfonyloxygruppen lassen sich in Form einer Säure der Formel $R_o-H$ auch durch Absorption, z.B. an Silikagel, Aluminiumoxyd, etc., und Elution (Chromatographie), aus Verbindungen der Formel V abspalten.

Die oben beschriebenen Säureabspaltungen werden in Abwesenheit, üblicherweise aber in Gegenwart eines Lösungsmittels, wie eines gegebenenfalls halogenierten Kohlenwasserstoffs aliphatischen, cycloaliphatischen oder aromatischen Charakters, wie Methylenchlorid, eines Niederalkanons, z.B. Aceton, oder Aethers, z.B. Tetrahydrofuran oder Dioxan, oder eines Lösungsmittelgemisches, inkl. eines wässrigen Gemisches, wenn notwendig unter Kühlen oder Erwärmen, bei -30° bis 150°, bevorzugt bei etwa -10° bis 60°, und/oder in einer Inertgas-, z.B. Stickstoffatmosphäre vorgenommen.

Verfahren d): Die Methylierung der Hydroxyiminomethylengruppe in einer Verbindung der Formel VI erfolgt auf an sich bekannte Weise durch Behandeln mit einem Methylierungsmittel. Im Ausgangsmaterial sind ausser der Hydroxyiminogruppe alle gegebenenfalls vorhandenen weiteren funktionellen Gruppen vorzugsweise geschützt.

Geeignete Methylierungsmittel sind beispielsweise Diazomethan, reaktionsfähige Ester von Methanol, wie Methylhalogenide, z.B. Methyliodid, Sulfonsäureester, z.B. Methansulfonsäure-, Trifluormethansulfonsäure- oder p-Toluolsulfonsäuremethylester, oder Schwefelsäureester, z.B. Dimethylsulfat, Dimethylacetale, z.B. 2,2-Dimethoxypropan, Orthoester, z.B. Orthoameisensäuretrimethylester, Trimethyloxoniumsalze, z.B. Trimethyloxoniumfluorantimonat, -hexachlorantimonat, -hexafluorphosphat oder -tetrafluorborat, Dimethoxycarboniumsalze, z.B. Dimethoxycarbonium-hexafluorphosphat, oder Dimethylhaloniumsalze, z.B. Dimethylbromonium-hexafluorantimonat, oder 3-Aryl-1-methyl-triazenverbindungen, z.B. 3-p-Tolyl-1-methyltriazen. Die Methylierung wird auf an sich bekannte Weise, üblicherweise in einem inerten Lösungsmittel, wie einem Aether, z.B. Dioxan oder Tetrahydrofuran, oder einem gegebenenfalls halogenierten Kohlenwasserstoff, wie Benzol, Chlorbenzol, Methylenchlorid, oder dergleichen, gegebenenfalls in Gegenwart geeigneter Kondensationsmittel, wie Basen oder Säuren, unter Kühlen oder Erwärmen, z.B. bei Temperaturen zwischen etwa -20° bis etwa 100°, durchgeführt.

Verfahren e): Die Veresterung der Carboxylgruppe in einer Verbindung der Formel VII oder eines reaktionsfähigen Derivates davon mit einem Alkohol der Formel VIII oder einem reaktionsfähigen Derivat davon wird auf an sich bekannte Weise durchgeführt.

Beispielsweise kann die freie Carboxylgruppe mit dem freien Alkohol in Gegenwart eines Kondensationsmittels, wie eines Carbodiimides, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, einer geeigneten Carbonylverbindung, beispielsweise Carbonyldiimidazol oder eines Isoxazoliniumsalzes, beispielsweise N-Aethyl-5-phenyl-isoxazolinium-3'sulfonat und N-tert.-Butyl-5-methyl-isoxazoliniumperchlorat, oder einer Acylaminoverbindung, z.B. 2-Aethoxy-1-äthoxycarbonyl-1,2-dihydrochinolin, verestert werden.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Zur Veresterung der freien Carboxylgruppe kann als reaktionsfähiges Derivat eines Alkoholes der Formel VIII ein Diazoessigester der Formel $N_2C(R_1)COOR_2$ eingesetzt werden, der, gegebenenfalls in situ, aus dem entsprechenden Glycolsäureester $H_2N-CH(R_1)-COOR_2$ und salpetriger Säure hergestellt und gegebenenfalls in Gegenwart einer Lewissäure, z.B. Bortrifluorid, in einem inerten Lösungsmittel, z.B. einem Aether, wie Diäthyläther, Dioxan oder Tetrahydrofuran, unter Kühlen, bei Raumtemperatur oder unter leichtem Erwärmen zur Anwendung gelangt.

Ein reaktionsfähiges Derivat eines Alkohols der Formel VIII ist auch ein solches, worin die Hydroxygruppe durch eine starke anorganische oder organische Säure zu einer reaktionsfähigen Gruppe verestert ist.

Entsprechende reaktionsfähige Gruppen sind insbesondere Halogen, z.B. Chlor, Brom oder bevorzugt Jod, ferner Sulfonyloxygruppen, wie Niederalkan- oder Arensulfonyloxygruppen, z.B. Methan-, Aethan-, Benzol- oder Toluolsulfonyloxygruppen, oder Halogensulfongruppen, z.B. die Chlorsulfongruppe, und dergleichen.

Die Veresterung der gegebenenfalls in Salzform vorliegenden 4-Carboxylgruppe mit diesen reaktionsfähigen Derivaten wird, auf an sich bekannte Weise, in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, eines N,N-Diniederalkyl-anilins, z.B. N,N-Dimethylanilin, eines cyclischen tertiären Amins, wie eines N-niederalkylierten Morpholins, z.B. N-Methylmorpholin, einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder einer quaternären Ammoniumbase, wie eines Tetraalkylammoniumhydroxids, -carbonats oder -hydrogencarbonats, z.B. worin Alkyl Methyl, Aethyl, Propyl, Isopropyl, Butyl oder dergleichen ist, oder eines Oxirans, beispielsweise eines niederen 1,2-Alkylenoxids, wie Aethylenoxid oder Propylenoxid, durchgeführt.

Bevorzugt wird die Carbonsäure der Formel VII zunächst in ein Salz einer der genannten organischen oder anorganischen Basen, insbesondere in das Natriumsalz, übergeführt und dieses mit einer reaktionsfähigen Verbindung der Formel VIII umgesetzt.

Ein reaktionsfähiges Derivat einer Verbindung der Formel VIII kann auch <u>in situ</u> hergestellt werden. Beispielsweise kann man eine Verbindung der Formel VIII, worin die Hydroxygruppe durch Chlor ersetzt ist, durch Behandeln mit Natriumjodid in einem Lösungsmittel, z.B. in Aceton oder Acetonitril, in eine Verbindung der Formel VIII überführen

worin die Hydroxygruppe durch Jod ersetzt ist, oder man kann die Veresterung mit der genannten Chlorverbindung in Gegenwart von Natriumjodid durchführen.

Ein reaktionsfähiges, d.h. esterbildendes, funktionelles Derivat einer Säure der Formel VII, worin Am mit Vorteil eine geschützte Aminogruppe darstellt, ist in erster Linie ein Anhydrid einer solchen Säure, vorzugsweise ein gemischtes Anhydrid, z.B. ein solches mit einer anorganischen Säure, wie einer Halogenwasserstoffsäure, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure.

Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure.

Weitere reaktionsfähige Säurederivate einer Säure der Formel VII sind aktivierte Ester, wie Ester mit vinylogen Alkohlen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthalyliminoester.

Die Veresterung des Alkohols der Formel VIII mit einem Derivat der Säure VII, wie einem Anhydrid, insbesondere mit einem Säurehalogenid, wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder

Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt. Die reaktionsfähigen Ester, z.B. die 1-Benztriazolester werden auch in Gegenwart eines der genannten Carbodiimide, z.B. N,N-Dicyclohexylcarbodiimid, verwendet.

Die obigen Veresterungsreaktionen werden bevorzugt in einem inerten, vorzugsweise wasserfreien Lösungsmittel oder Lösungsmittelgemisch vorgenommen, beispielsweise in einem Carbonsäureamid, wie einem Formamid, z.B. Dimethylformamid, einem Sulfoxid, z.B. Dimethylsulfoxid oder flüssigem $SO_2$, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, bei Raumtemperatur , wenn notwendig, bei erniedrigter oder erhöhter Temperatur, etwa bei -40° bis etwa 100°, bevorzugt bei -10° bis +40°, und/oder in einer Inertgas-, z.B. Stickstoffatmosphäre.

In einer acylierenden Säure der Formel VII oder in einem Säurederivat davon kann eine geschützte Aminogruppe auch in ionischer Form vorliegen, d.h. das Ausgangsmaterial der Formel VII kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, in situ gebildet werden. So erhält man z.B. ein gemischtes Anhydrid durch Behandeln einer Säure der Formel VII mit entsprechend geschützten funktionellen Gruppen, oder eines geeigneten Salzes davon, wie eines Ammoniumsalzes, z.B. mit

einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B, Trichloracetylchlorid, oder einem Halbester eines Kohlensäurehalbhalogenides, z.B. Chlorameisensäureäthylester oder -isobutylester oder mit $SOCl_2$, und verwendet das so erhältliche gemischte Anhydrid ohne Isolierung.

Verfahren f): Die Veresterung der Carboxylgruppe in einer Verbindung der Formel IX oder eines reaktionsfähigen Derivates davon mit einem Alkohol der Formel $R_2$-OH oder einem reaktionsfähigen Derivat davon kann auf an sich bekannte Weise analog wie im Verfahren e) beschrieben durchgeführt werden.

Verfahren g): Eine geschützte Aminogruppe Am setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Brom-niederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino, Formylamino oder 2-Acyl-niederalk-1-en-1-yl-amino, z.B. durch Behandeln mit einer

Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silyl- oder Stannylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silyläthoxycarbonyl geschützte Aminogruppe kann auch durch Behandeln mit einem Fluoridanionen liefernden Salz der Fluorwasserstoffsäure, in die freie Aminogruppe übergeführt werden. Eine Phosphor-, Phosphon- oder Phosphin-amidogruppe kann z.B. durch Behandeln mit einer phosphorhaltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, in die freie Aminogruppe übergeführt werden.

Die beschriebenen Spaltungsreaktionen werden im übrigen unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

Bevorzugt werden selektive Abspaltungsreaktionen durchgeführt, d.h. solche bei denen die Estergruppierung und die Methoxyiminogruppe entweder gar nicht oder nur wenig angegriffen werden. Eine solche bevorzugte Methode ist die acidolytische Abspaltung einer tert.-Niederalkoxycarbonylgruppe, insbesondere der tert.-Butoxycarbonylgruppe, mittels einer geeigneten Säure, insbesondere Trifluoressigsäure, gegebenenfalls in einem inerten Lösungsmittel, wie einem gegebenenfalls halogenierten Kohlenwasserstoff, z.B. Methylenchlorid, bei Temperaturen von etwa -20° bis etwa 50°, bevorzugt bei 0° bis Raumtemperatur.

Säureadditionssalze von Verbindungen der Formel I, worin Am eine freie Aminogruppe bedeutet, erhält man in üblicher Weise, z.B. durch Behandeln mit einer Säure oder einem geeigneten Anionenaustauschreagens.

Erhaltene Gemische von Isomeren können nach an sich bekannten Methoden, in die einzelnen Isomeren getrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren. Erhaltene Racemate können in üblicher Weise, gegebenenfalls nach Einführen von geeigneten salzbildenden Gruppierungen, z.B. durch Bilden eines Gemisches von diastereomeren Salzen und Ueberführung der optisch aktiven Salze in die freie Verbindung oder durch fraktioniertes Kristallisieren aus optisch aktiven Lösungsmitteln, in die Antipoden getrennt werden.

Die Verfahren umfassen auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbindungen, gebildet werden.

Die Ausgangsverbindungen der Formeln II bis VIII und X sind bekannt oder können analog bekannten Verfahren hergestellt werden.

Die Ausgangsverbindungen der Formel IX sind neu und ebenfalls Gegenstand der vorliegenden Erfindung. Sie können in Analogie zu einem der Verfahren a) bis e) oder f) hergestellt werden, wobei die Carboxylgruppe gegebenenfalls in geschützter Form vorliegen und auf konventionelle Weise abgespalten werden kann.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich zur enteralen Verabreichung eignen. Zur enteralen Verabreichung verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Suppositorien sind in erster Linie Fettemulsionen oder -suspensionen.

Die vorliegenden pharmazeutischen Präparate, die, wenn erwünscht, weitere pharmakologisch wertvolle Stoffe enthalten können, werden in an sich bekannter Weise, z.B. mittels konventioneller Mischungs-, Lösungs-, oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion, Zustand des infizierten Organismus verwendet man tägliche Dosen von etwa 0,25 g bis etwa 2 g p.o. zur Behandlung von Warmblütern von etwa 70 kg Gewicht.

Die folgenden Beispiele dienen zur Illustration der Erfindung; vor- und nachstehend werden Temperaturen in Celsiusgrade angegeben. DC = Dünnschichtchromatogramm auf Silikagelplatten.

Beispiel 1: Ceftizoxime-(ℓ)-bornyloxycarbonylmethylester

Eine Suspension von 2,03 g (5 mMol) Ceftizoxime-natriumsalz in 22 ml N,N-Dimethylformamid wird zusammen mit 3,23 g (10 mMol) (ℓ)-Bornyloxycarbonylmethyljodid 2 1/2 Stunden bei 22° gerührt. Man verdünnt mit Essigsäureäthylester und wäscht mit Wasser, 1-normaler Natriumbicarbonat-Lösung und gesättigter Kochsalzlösung. Der eingedampfte Extrakt wird an 150 g Kieselgel 60, reinst (Merck) chromatographiert. Die mit Toluol-Essigsäureäthylester 1:1 eluierten Fraktionen 55-85 (je 25 ml) enthalten als Eindampfrückstand ein Produkt, das mehrmals mit Diäthyläther digeriert wird. Man erhält den Ceftizoxime-(ℓ)-bornyloxycarbonylmethylester. F. 140-145°; DC (Essigsäureäthylester): Rf 0.35.

Das Ausgangsmaterial kann wie folgt hergestellt werden:

Eine Mischung von 50,0 g (64,8 mMol) (ℓ)-Borneol und 28,9 ml (71,3 mMol) Chloressigsäurechlorid wird 30 min bei 60° gerührt. Die Lösung wird auf ein Gemisch aus Eiswasser und Diäthyläther gerührt und die abgetrennte organische Phase mit gesättigter Natriumbicarbonatlösung gewaschen. Trocknen über Natriumsulfat und Eindampfen geben (ℓ)-Bornyloxycarbonylmethylchlorid.

5,0 g (20 mMol) dieses Chlorids und 4,3 g (30 mMol) Natriumjodid werden in 23 ml Aceton 2 Stunden bei 22° gerührt. Man verdünnt mit Essigsäureäthylester und wäscht mit kaltem Wasser sowie 0,1-normaler Natriumthiosulfat-Lösung. Trocknen über Natriumsulfat und Eindampfen geben das (ℓ)-Bornyloxycarbonylmethyljodid.

Beispiel 2: Ceftizoxime-(ℓ)-bornyloxycarbonylmethylester

Eine Lösung von 4,41 g (10 mMol) Ceftizoxime-carboxymethylester, 1,6 ml (20 mMol) Pyridin, 1,85 ml (12 mMol) (ℓ)-Borneol, 10 ml Acetonitril und 15 ml N,N-Dimethylformamid wird bei 0° mit 340 mg (2,5 mMol) 1-Hydroxybenztriazol und 2,25 g (11 mMol) Dicyclohexylcarbodiimid versetzt und 23 Stunden bei 0° stehen gelassen. Die Reaktionslösung wird klarfiltriert und wie im Beispiel 1 aufgearbeitet. Man erhält Ceftizoxime-(ℓ)-bornyloxycarbonylmethylester. F. 143-146° (identisch mit Produkt von Beispiel 1).

Das Ausgangsmaterial kann wie folgt hergestellt werden:

Eine Suspension von 37,0 g (61 mMol) Ceftizoxime-benzhydryloxycarbonylmethylester in 500 ml Methylenchlorid wird bei 0° mit 34 ml Anisol, dann mit 95 ml Trifluoressigsäure versetzt. Man rührt 1 Stunde bei 0°. Die Reaktionslösung wird im Vakuum eingedampft und der Rückstand in 1 l Diäthyläther aufgeschüttelt. Das abfiltrierte Rohprodukt wird in 200 ml Wasser suspendiert und durch Zugabe von 200 ml 1-normaler Natriumbicarbonat-Lösung gelöst. Man filtriert klar, tropft bei 0° 100 ml 2-normale Salzsäure zu und erhält Ceftizoximecarboxymethylester.

Beispiel 3: Ceftizoxime-cyclohexyloxycarbonylmethylesterhydrochlorid

Eine Lösung von 4,41 g Ceftizoxime-carboxymethylester in 60 ml Acetonitril und 150 ml N,N-Dimethylformamid wird mit 1,2 g 4-Dimethylaminopyridin, 1,27 ml Cyclohexanol, 2,2 g N,N-Dicyclohexylcarbodiimid und 340 mg 1-Hydroxybenzotriazol versetzt und 17 Stunden bei 22° gerührt. Man dampft im Vakuum das Acetonitril ab und verdünnt mit 1,5 l Essigsäureäthylester. Nach Abfiltrieren wird die Lösung mit 0,3-molarer, wässriger Dikaliumhydrogenphosphat-Lösung gewaschen. Die organische Phase wird über Magnesiumsulfat getrocknet, mit Aktivkohle entfärbt und eingedampft. Der ölige Rückstand wird in 20 ml Methylenchlorid aufgenommen, mit 33 ml 0,65-normaler Hydrogenchlorid-Lösung in Methylenchlorid versetzt, und die Mischung auf 400 ml Aether gerührt. Dabei fällt das Produkt aus. Durch Filtration erhält man das Ceftizoxime-cyclohexyloxycarbonylmethylester-Hydrochlorid. DC (Kieselgel/Toluol-Aceton 2:3): Rf = 0,59.

Beispiel 4: Ceftizoxime-(ℓ)-menthyloxycarbonylmethylesterhydrochlorid
Werden 4,41 g Ceftizoxime-carboxymethylester analog Beispiel 3 mit 1,88 g ℓ-Menthol umgesetzt und das Produkt wie dort aufgearbeitet, so wird das Ceftizoxime-ℓ-menthyloxycarbonylmethylesterhydrochlorid erhalten.
DC (Kieselgel/Toluol-Aceton 2:2): Rf = 0,51.

Beispiel 5: In Analogie zu den vorstehenden Beispielen können ausgehend von Ceftizoxime und den entsprechenden Chloriden, bzw. Jodiden, auch die folgenden Verbindungen hergestellt werden:

Ceftizoxime-endo-norbornyloxycarbonylmethylester und
Ceftizoxime-exo-norbornyloxycarbonylmethylester,

in optisch aktiver oder in racemischer Form, sowie die pharmazeutischen Salze davon.

In den folgenden Beispielen wird als Wirksubstanz der Ceftizoxime-(ℓ)-bornyloxycarbonylmethylester verwendet.

Beispiel 6: Kapseln, enthaltend 0,250 g Wirksubstanz werden wie folgt hergestellt:

Zusammensetzung (für 100'000 Kapseln):

| | |
|---|---|
| Wirksubstanz | 25'000 g |
| Weizenstärke | 2'500 g |
| Magnesiumstearat | 1'000 g |

Die Wirksubstanz, die Weizenstärke und das Magnesiumstearat werden gut miteinander vermischt und in Kapseln Nr. 1 abgefüllt.

Beispiel 7: Kapseln enthaltend 0,5 g Wirksubstanz werden wie folgt hergestellt:

Zusammensetzung (für 2000 Kapseln):

| | |
|---|---|
| Wirksubstanz | 1000.00 g |
| Polyvinylpyrrolidon | 15.00 g |
| Maisstärke | 115.00 |
| Magnesiumstearat | 20.00 g |

Man befeuchtet die Wirksubstanz mit 300 ml einer Lösung des Polyvinylpyrrolidons in 95 %igem Aethanol, treibt das Gemisch durch ein Sieb mit 3 mm Maschenweite und trocknet das Granulat unter vermindertem Druck bei 40-50°. Man siebt durch ein Sieb mit 0,8 mm Maschenweite, gibt die Maisstärke und das Magnesiumstearat zu, vermischt und füllt das Gemisch in Steckkapseln (Grösse 0) ab.

Beispiel 8: Tabletten, enthaltend 250 mg Wirksubstanz werden wie folgt hergestellt:

Zusammensetzung (für 1 Tablette):

| | |
|---|---|
| Wirksubstanz | 250 mg |
| Mikrokristalline Cellulsoe | 80 mg |
| Natrium-carboxymethyl-stärke | 10 mg |
| Magnesiumstearat | 3 mg |
| Talk | 7 mg |
| | 350 mg |

Die Wirksubstanz wird mit den Zusatzstoffen homogen vermischt und zu Tabletten gepresst.

Zur Herstellung von Filmdragées werden die Tabletten je mit 1 mg wässrigem Lack überzogen.

Anstelle von Natriumcarboxymethyl-stärke kann auch Natrium-carboxymethylcellulsoe eingesetzt werden.

In den Beispielen 6 bis 8 kann als Wirksubstanz auch ein anderer in den vorstehenden Beispielen genannter Ceftizoxime-cycloalkyloxycarbonylmethylester oder ein Salz dieser Verbindungen, z.B. das Hydrochlorid oder Hydrobromid, eingesetzt werden.

Patentansprüche

(für alle benannten Länder ausser Oesterreich)

1. Verbindungen der Formel

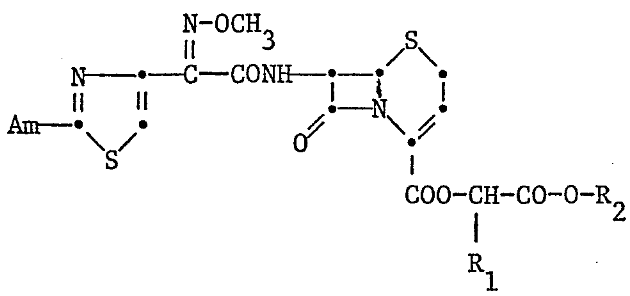

I,

worin $R_1$ Wasserstoff oder Niederalkyl, $R_2$ einen cycloaliphatischen Rest und Am eine gegebenenfalls geschützte Aminogruppe bedeuten und Säureadditionssalze von solchen Verbindungen mit salzbildender Gruppe.

2. Eine Verbindung der Formel I, nach Anspruch 1, worin Am eine freie Aminogruppe ist und Säureadditionssalze davon.

3. Eine Verbindung der Formel I, nach Anspruch 1, worin $R_1$ Wasserstoff ist und Säureadditionssalze von solchen Verbindungen mit salzbildender Gruppe.

4. Eine Verbindung der Formel I, nach Anspruch 1, worin Am eine freie Aminogruppe, $R_1$ Wasserstoff und $R_2$ gegebenenfalls durch Niederalkyl ein- bis dreifach substituiertes Cycloalkyl oder Bicycloalkyl bedeutet, und ihre pharmazeutisch annehmbaren Salze, sowie die entsprechenden Verbindungen mit geschützten funktionellen Gruppen.

5. Eine Verbindung der Formel I, nach Anspruch 1, worin Am eine freie Aminogruppe, $R_1$ Wasserstoff und $R_2$ gegebenenfalls durch Methyl und/oder Isopropyl ein- bis dreifach substituiertes Cyclohexyl, Bicyclohexyl oder Bicycloheptyl bedeutet, und ihre pharmazeutisch annehmbaren Salze, sowie die entsprechenden Verbindungen mit geschützten funktionellen Gruppen.

6. Eine Verbindung der Formel I, nach Anspruch 1, worin Am eine freie Aminogruppe, $R_1$ Wasserstoff und $R_2$ Cyclohexyl, (-ℓ)-Menthyl, (-ℓ)-Bornyl oder endo- oder exo-Norbornyl bedeutet, und ihre pharmazeutisch annehmbaren Salze, sowie die entsprechenden Verbindungen mit geschützten funktionellen Gruppen.

7. Eine Verbindung der Formel I, nach Anspruch 1, worin Am die freie Aminogruppe, $R_1$ Wasserstoff und $R_2$ (-ℓ)-Bornyl, Cyclohexyl oder (-ℓ)-Menthyl bedeuten, und ihre pharmazeutisch annehmbaren Salze.

8. Eine Verbindung der Formel I, nach Anspruch 1, worin Am die freie Aminogruppe, $R_1$ Wasserstoff und $R_2$ (-ℓ)-Bornyl bedeuten, und ihre pharmazeutisch annehmbaren Salze.

9. Eine Verbindung der Formel I, nach Anspruch 1, worin Am die freie Aminogruppe, $R_1$ Wasserstoff und $R_2$ Cyclohexyl bedeuten, und ihre pharmazeutisch annehmbaren Salze.

10. Eine Verbindung der Formel I, nach Anspruch 1, worin Am die freie Aminogruppe, $R_1$ Wasserstoff und $R_2$ (-ℓ)-Menthyl bedeuten und ihre pharmazeutisch annehmbaren Salze.

11. Das Hydrochlorid einer Verbindung der Formel I gemäss einem der Ansprüche 1-10, worin Am die freie Aminogruppe ist.

12. Pharmazeutische Präparate enthaltend eine der in den Ansprüchen 1-11 beschriebenen Verbindungen.

13. Verbindungen gemäss einem der Ansprüche 1-11 zur Verwendung als orale Antibiotika.

14. Verwendung von Verbindungen gemäss einem der Ansprüche 1-11 und ihrer pharmazeutisch annehmbaren Salze zur Herstellung pharmazeutischer Präparate.

15. Verfahren zur Herstellung von Verbindungen der Formel

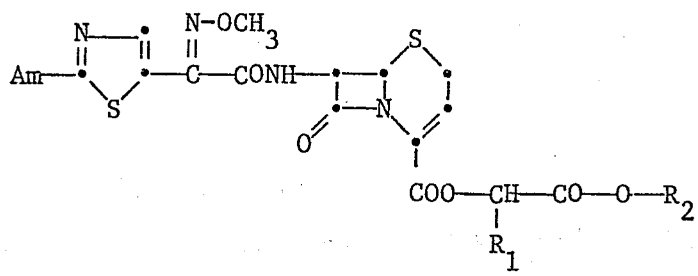

I,

worin $R_1$ Wasserstoff oder Niederalkyl, $R_2$ einen cycloaliphatischen Rest und Am eine gegebenenfalls geschützte Aminogruppe bedeuten und der Säureadditionssalze von solchen Verbindungen, dadurch gekennzeichnet, dass man

a) die 7β-Aminogruppe in einer Verbindung der Formel

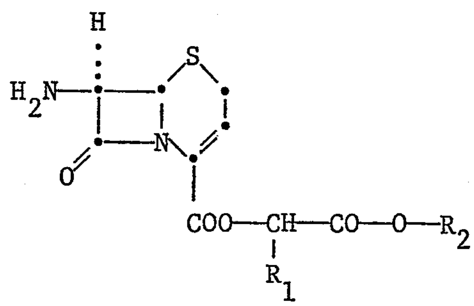

II,

worin die Aminogruppe gegebenenfalls durch eine die Acylierung erlaubende Gruppe substituiert ist und worin $R_1$ und $R_2$ die unter Formel I gegebenen Bedeutungen haben, durch Behandeln mit einem den Acylrest einer Carbonsäure der Formel

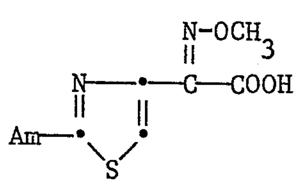

III

einführenden Acylierungsmittel, worin Am die unter Formel I angegebenen Bedeutungen hat, acyliert, oder

b) eine Verbindung der Formel

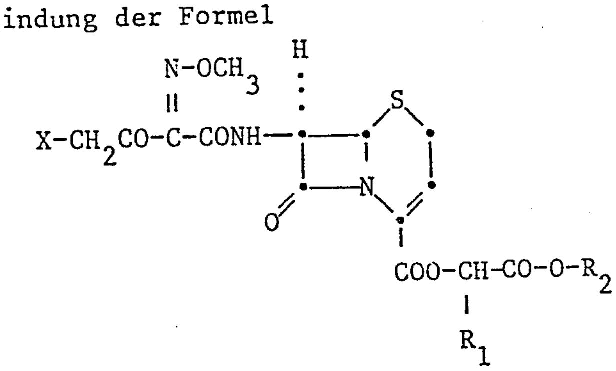

IV ,

worin X Halogen bedeutet, $R_1$ und $R_2$ die unter Formel I genannten Bedeutungen haben, mit einem Thioharnstoff der Formel $Am-CS-NH_2$ oder einem Salz davon kondensiert, oder

c) aus einer Verbindung der Formel

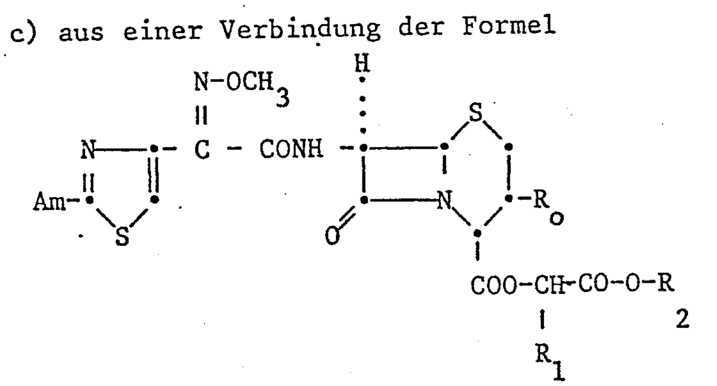

V ,

worin Am, $R_1$ und $R_2$ die unter Formel I angegebenen Bedeutungen haben und $R_o$ Hydroxy, verestertes Hydroxy oder gegebenenfalls substituiertes Amino ist, eine Gruppe $H-R_o$ abspaltet, oder

d) in einer Verbindung der Formel

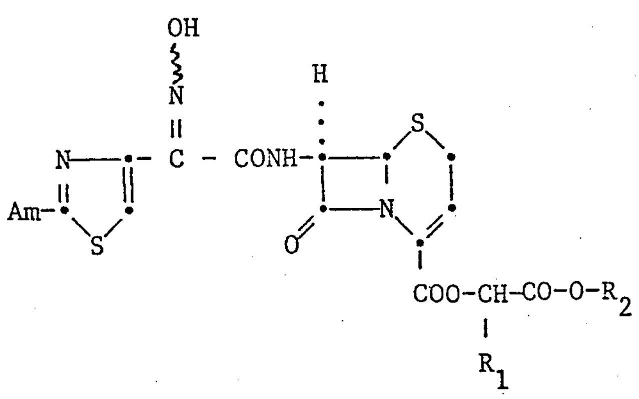

VI ,

worin Am, $R_1$ und $R_2$ die unter Formel I gegebenen Bedeutungen haben, die Hydroxyiminogruppe methyliert, oder

e) in einer Verbindung der Formel

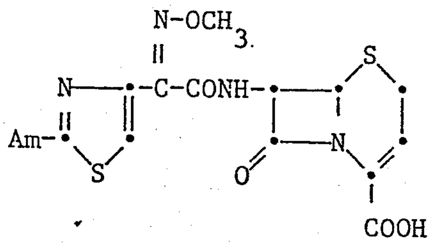

VII ,

worin Am die unter Formel I gegebene Bedeutung hat, die Carboxylgruppe oder ein reaktionsfähiges Derivat davon, mit einem Alkohol der Formel

$$HO\text{-}\underset{\displaystyle R_1}{\underset{|}{CH}}\text{-}CO\text{-}O\text{-}R_2 \qquad \text{VIII ,}$$

worin $R_1$ und $R_2$ die unter Formel I angegebenen Bedeutungen haben, oder einem reaktionsfähigen Derivat davon, in eine veresterte Carboxylgruppe überführt, oder

f) in einer Verbindung der Formel

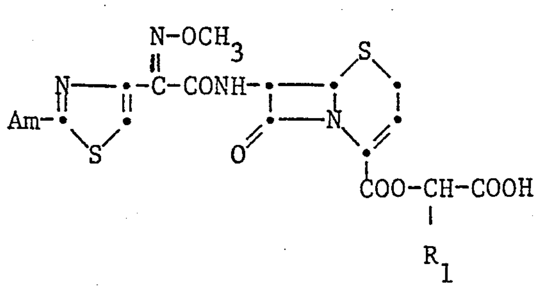

IX ,

worin Am und $R_1$ die unter Formel I gegebenen Bedeutungen haben, die Carboxylgruppe mit einem Alkohol der Formel $R_2$-OH (X) oder einem reaktionsfähigen Derivat davon in eine veresterte Carboxylgruppe überführt, und/oder

g) zur Herstellung einer Verbindung der Formel I, worin Am eine freie Aminogruppe ist und $R_1$ und $R_2$ die unter Formel I gegebenen Bedeutungen haben, aus einer Verbindung der Formel I, worin Am eine geschützte Aminogruppe bedeutet, und $R_1$ und $R_2$ die unter Formel I gegebenen Bedeutungen haben, die Aminoschutzgruppe abspaltet und durch Wasserstoff ersetzt, wenn erwünscht, eine erhaltene Verbindung, worin Am eine freie Aminogruppe ist, in ein Salz oder ein erhaltenes Salz in eine freie Verbindung oder in ein anderes Salz überführt und/oder, wenn erwünscht, ein erhaltenes Isomerengemisch in die einzelnen Isomeren auftrennt.

16. Die nach einem Verfahren gemäss Anspruch 15 erhältlichen Verbindungen.

Patentansprüche
(für Oesterreich)

1. Verfahren zur Herstellung von Verbindungen der Formel

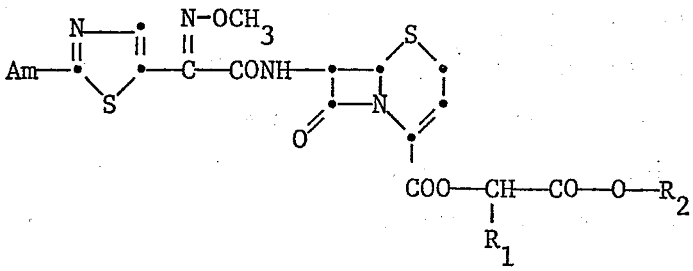

I,

worin $R_1$ Wasserstoff oder Niederalkyl, $R_2$ einen cycloaliphatischen Rest und Am eine gegebenenfalls geschützte Aminogruppe bedeuten und der Säureadditionssalze von solchen Verbindungen, dadurch gekennzeichnet, dass man

a) die 7β-Aminogruppe in einer Verbindung der Formel

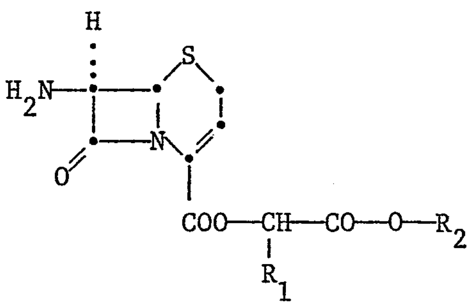

II,

worin die Aminogruppe gegebenenfalls durch eine die Acylierung erlaubende Gruppe substituiert ist und worin $R_1$ und $R_2$ die unter Formel I gegebenen Bedeutungen haben, durch Behandeln mit einem den Acylrest einer Carbonsäure der Formel

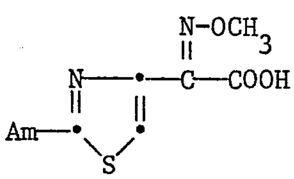

III

einführenden Acylierungsmittel, worin Am die unter Formel I angegebenen Bedeutungen hat, acyliert, oder

b) eine Verbindung der Formel

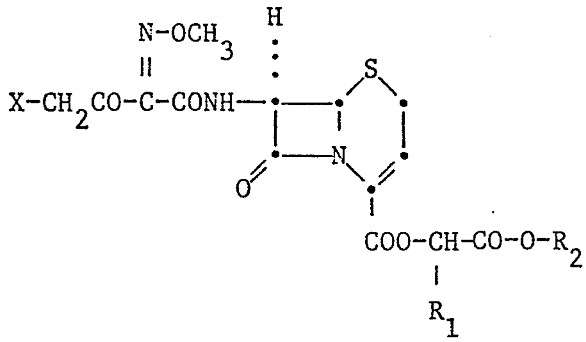

IV ,

worin X Halogen bedeutet, $R_1$ und $R_2$ die unter Formel I genannten Bedeutungen haben, mit einem Thioharnstoff der Formel $Am-CS-NH_2$ oder einem Salz davon kondensiert, oder

c) aus einer Verbindung der Formel

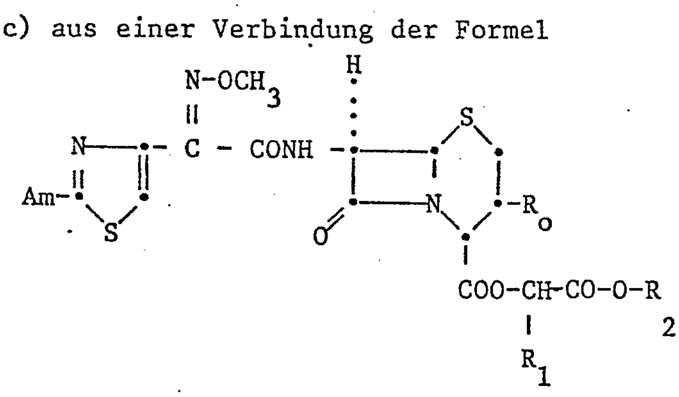

V ,

worin Am, $R_1$ und $R_2$ die unter Formel I angegebenen Bedeutungen haben und $R_o$ Hydroxy, verestertes Hydroxy oder gegebenenfalls substituiertes Amino ist, eine Gruppe $H-R_o$ abspaltet, oder

d) in einer Verbindung der Formel

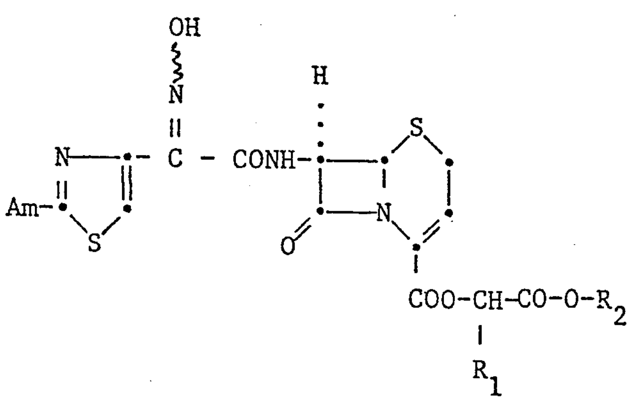

VI ,

worin Am, $R_1$ und $R_2$ die unter Formel I gegebenen Bedeutungen haben, die Hydroxyiminogruppe methyliert, oder

e) in einer Verbindung der Formel

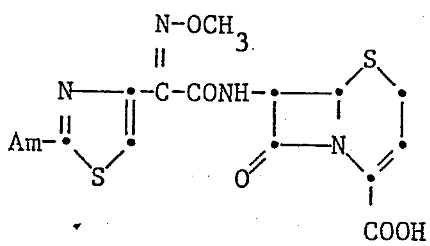

VII ,

worin Am die unter Formel I gegebene Bedeutung hat, die Carboxylgruppe oder ein reaktionsfähiges Derivat davon, mit einem Alkohol der Formel

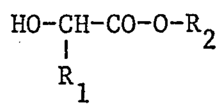

VIII ,

worin $R_1$ und $R_2$ die unter Formel I angegebenen Bedeutungen haben, oder einem reaktionsfähigen Derivat davon, in eine veresterte Carboxylgruppe überführt, oder

f) in einer Verbindung der Formel

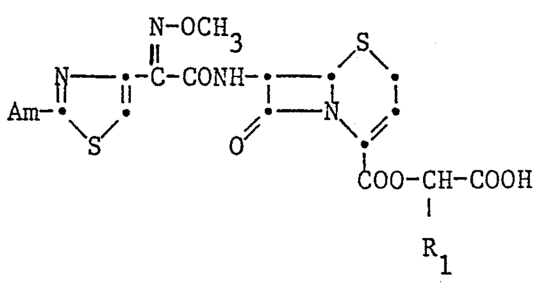

IX ,

worin Am und $R_1$ die unter Formel I gegebenen Bedeutungen haben, die Carboxylgruppe mit einem Alkohol der Formel $R_2$-OH (X) oder einem reaktionsfähigen Derivat davon in eine veresterte Carboxylgruppe überführt, und/oder

g) zur Herstellung einer Verbindung der Formel I, worin Am eine freie Aminogruppe ist und $R_1$ und $R_2$ die unter Formel I gegebenen Bedeutungen haben, aus einer Verbindung der Formel I, worin Am eine geschützte Aminogruppe bedeutet, und $R_1$ und $R_2$ die unter Formel I gegebenen Bedeutungen haben, die Aminoschutzgruppe abspaltet und durch Wasserstoff ersetzt, wenn erwünscht, eine erhaltene Verbindung, worin Am eine freie Aminogruppe ist, in ein Salz oder ein erhaltenes Salz in eine freie Verbindung oder in ein anderes Salz überführt und/oder, wenn erwünscht, ein erhaltenes Isomerengemisch in die einzelnen Isomeren auftrennt.

2. Verfahren gemäss Anspruch 1 zur Herstellung einer Verbindung der Formel I, worin Am eine freie Aminogruppe ist und Säureadditionssalze davon.

3. Verfahren gemäss Anspruch 1 zur Herstellung einer Verbindung der Formel I, worin $R_1$ Wasserstoff ist und Säureadditionssalze von solchen Verbindungen mit salzbildender Gruppe.

4. Verfahren gemäss Anspruch 1 zur Herstellung einer Verbindung der Formel I, worin Am eine freie Aminogruppe, $R_1$ Wasserstoff und $R_2$ gegebenenfalls durch Niederalkyl ein- bis dreifach substituiertes Cycloalkyl oder Bicycloalkyl bedeutet, und ihre pharmazeutisch annehmbaren Salze, sowie die entsprechenden Verbindungen mit geschützten funktionellen Gruppen.

5. Verfahren gemäss Anspruch 1 zur Herstellung einer Verbindung der Formel I, worin Am eine freie Aminogruppe, $R_1$ Wasserstoff $R_2$ gegebenenfalls durch Methyl und/oder Isopropyl ein- bis dreifach substituiertes Cyclohexyl, Bicyclohexyl oder Bicycloheptyl bedeutet, und ihre pharmazeutisch annehmbaren Salze, sowie die entsprechenden Verbindungen mit geschützten funktionellen Gruppen.

6. Verfahren gemäss Anspruch 1 zur Herstellung einer Verbindung der Formel I, worin Am eine freie Aminogruppe, $R_1$ Wasserstoff und $R_2$ Cyclohexyl, (ℓ)-Menthyl, (ℓ)-Bornyl oder endo- oder exo-Norbornyl bedeutet, und ihre pharmazeutisch annehmbaren Salze, sowie die entsprechenden Verbindungen mit geschützten funktionellen Gruppen.

7. Verfahren gemäss Anspruch 1 zur Herstellung einer Verbindung der Formel I, worin Am die freie Aminogruppe, $R_1$ Wasserstoff und $R_2$ (ℓ)-Bornyl, Cyclohexyl oder (ℓ)-Menthyl bedeuten, und ihre pharmazeutisch annehmbaren Salze.

8. Verfahren gemäss Anspruch 1 zur Herstellung einer Verbindung der Formel I, worin Am die freie Aminogruppe, $R_1$ Wasserstoff und $R_2$ (ℓ)-Bornyl bedeuten, und ihre pharmazeutisch annehmbaren Salze.

9. Verfahren gemäss Anspruch 1 zur Herstellung einer Verbindung der Formel I, worin Am die freie Aminogruppe, $R_1$ Wasserstoff und $R_2$ Cyclohexyl bedeuten, und ihre pharmazeutisch annehmbaren Salze.

10. Verfahren gemäss Anspruch 1 zur Herstellung einer Verbindung der Formel I, worin Am die freie Aminogruppe, $R_1$ Wasserstoff und $R_2$ (ℓ)-Menthyl bedeuten und ihre pharmazeutisch annehmbaren Salze.

11. Verfahren gemäss einem der Ansprüche 1-10 zur Herstellung des Hydrochlorides einer Verbindung der Formel I, worin Am die freie Aminogruppe ist.